# EUROPEAN PATENT APPLICATION

(11) **EP 1 604 986 A1**
(43) Date of publication of application: **14.12.2005**
(21) Application number: 04719048.3
(22) Date of filing: 10.03.2004
(51) Int. Cl.: C07D 311/22

(54) **NOVEL CRYSTAL OF N- 3-(FORMYLAMINO)-4-OXO-6-PHENOXY-4H-CROME NE-7-YL METHANESULFONAMIDE**

(30) Priority: 14.03.2003 JP 2003069241
(71) Applicant: TOYAMA CHEMICAL CO., LTD., Tokyo 160-0023 (JP)
(72) Inventor: KAJITA, Tetsuya, Toyama 933-0874 (JP); INABA, Takihiro, Namerikawa-shi, Toyama 936-0056 (JP)
(74) Representative: Hartz, Nikolai
(86) International application number: PCT/JP2004/003057
(87) International publication number: WO 2004/080991

(57) **Abstract**

The crystal of N-[3-(formylamino)-4-oxo-6-phenoxy-4H-chromen-7-yl]methanesulfonamide having peaks at the positions of 6.00±0.05°, 10.54±0.05°, 17.42±0.08°, 18.39±0.08°, 20.13±0.10° and 23.01±0.10° on 2θ of diffraction angle in powder X-ray diffraction pattern of present invention is low hygroscopic, stable under a high humidity condition, and is useful as a drug substance of excellent anti-inflammatory agent.

## Description

### TECHNICAL FIELD

The present invention relates to novel crystals of N-(3-(formylamino)-4-oxo-6-phenoxy-4H-chromen-7-yl]methanesulfonamide, a useful anti-inflammatory agent which has excellent anti-inflammatory activity, antipyretic analgesic activity, antiarthritic activity and antiallergic activity, and of its hydrate.

### BACKGROUND ART

In Japanese Patent Laid-Open No. 2-49778, it is described that N-[3-(formylamino)-4-oxo-6-phenoxy-4H-chromen-7-yl]methanesulfonamide(hereinafter abbreviated as "T-614") has excellent anti-inflammatory activity, antipyretic analgesic activity, antiarthritic activity and antiallergic activity, and that it is useful as an anti-inflammatory agent. In addition, the manufacturing process of crystallization of T-614 out of the hydrous acetone solution is mentioned in Japanese Patent Laid-Open No. 5-97840 and Japanese Patent Laid-Open No. 5-125072. However, since the crystal of T-614 (hereinafter abbreviated as "T-614α") produced by the method mentioned in these patents is easily hygroscopic and followed by increasing in moisture, the quality control of T-614α was difficult. The stable crystal of T-614 which has low hygroscopicity under high humidity condition has been completely unknown until now.

### DISCLOSURE OF THE INVENTION

Under these conditions, the inventors of the present invention directed tremendous research effort toward coming up to the above expectation and hope, and they have found the stable crystals of T-614 described as follows: the crystal of T-614(hereinafter abbreviated as "T-614β") having peaks at the positions of 6.00±0.05°, 10.54±0.05°, 17.42±0.08°, 18.39±0.08°, 20.13±0.10° and 23.01±0.10° on 2θ of diffraction angle in powder X-ray diffraction pattern, the crystal of T-614(hereinafter abbreviated as "T-614γ") having peaks at the positions of 11.28±0.05°, 17.44±0.08°, 18.12±0.08°, 19.78±0.08°, 21.70±0.10°, 22.68±0.10° and 24.18±0.10° on 2θ of diffraction angle in powder X-ray diffraction pattern, and the crystal of T-614 monohydrate (hereinafter abbreviated as "T-614 hydrate") having peaks at the positions of 9.31±0.05°, 9.81±0.05°, 15.35±0.08°, 19.48±0.08°, 20.80±0.10°, 24.48±0.10° and 24.84±0.10° on 2θ of diffraction angle in powder X-ray diffraction pattern, respectively which has low hygroscopicity and shows further no increase in moisture under high humidity condition, and also each of them is superior as drug substance.

In particular, because T-614β has the property of small specific volume and hard to be charged with electricity, T-614β is easy to be handled.

Incidentally, the existence of these crystals in the present invention has been completely unknown until now, and has not been disclosed at all in the above-mentioned patent gazettes.

Now, a manufacturing process of the compounds of the present invention is described.

For example, T-614β can be produced according to Manufacturing Process 1-3.

### [Manufacturing Process 1]

T-614β can be produced by recrystallizing T-614α from 2-butanone, benzene, toluene or xylene. These solvents may be used in combination.

### [Manufacturing Process 2]

T-614β can be produced by stirring T-614α, T-614γ or T-614 hydrate in 2-butanone, benzene, toluene or xylene. These solvents may be used in combination. The amount of solvent used is not limited in particular, but it may be preferably 5-200 times the amount of the solute. Stirring time is not limited in particular, but it may be preferably 1-72 hours. Stirring temperature is not limited in particular, but it may be preferably 10-30°C.

### [Manufacturing Process 3]

T-614β can be produced by heating T-614α, T-614γ or T-614 hydrate in the presence or absence of solvent. Such as benzene, toluene, xylene or water is selected as the solvent for use. These solvents may be used in combination. As for heating temperature, in case of the presence of solvent it is preferably the boiling point of such solvent, and in case of the absence of solvent it is preferably 140-200°C.

For example, T-614γ can be produced according to Manufacturing Process 4.

### [Manufacturing Process 4]

T-614γ can be produced by stirring T-614 hydrate in acetonitrile. The amount of acetonitrile used is not limited in particular, but it may be preferably 5-200 times the amount of the solute. Stirring time is not limited in particular, but it may be preferably 1-48 hours. Stirring temperature is not limited in particular, but it may be preferably 10-30°C.

For example, T-614 hydrate can be produced according to Manufacturing Process 5.

### [Manufacturing Process 5]

T-614 hydrate can be produced by stirring T-614α, T-614β or T-614γ in water. The amount of water used is not limited in particular, but preferably it may be 5-200 times the amount of the solute. Stirring time is not limited in particular, but it may be preferably 0.5-10 days. Stirring temperature is not limited in particular, but it may be preferably 10-30°C.

When the compound of the present invention (T-614β, T-614γ or T-614 hydrate) is used as drugs, such compound can be used individually or with mixture. In addition, the compound of the present invention may be mixed appropriately with pharmaceutical additives that are used in pharmaceutical preparation, such as excipient, carrier and diluent. According to conventional method, they can be orally or parenterally administered in a dosage form of tablets, capsules, powders, syrups, granules, pills, suspensions, emulsions, solutions, powder preparations, suppositories or ointments.

Furthermore, the compound of the present invention may be used as the mixture with T-614α.

### BRIEF DESCRIPTION OF DRAWINGS

FIG. 1
   A figure of powder X-ray diffraction pattern for T-614β
FIG. 2
   A figure of powder X-ray diffraction pattern for T-614γ
FIG. 3
   A figure of powder X-ray diffraction pattern for T-614 hydrate
FIG. 4
   A figure of powder X-ray diffraction pattern for T-614α

### BEST MODE FOR CARRYING OUT THE INVENTION

The present invention is explained in detail based on the following Test Examples and Examples; however the present invention is not intended to be limited to these.

### Test Example 1 Influence of humidity

After being allowed to stand the compounds of the present invention (T-614β, T-614γ and T-614 hydrate) and the compound disclosed as Example 5 in Japanese Patent Laid-Open No. 5-97840 (T-614α) under the relative humidity 22, 51, 75 and 93%RH for 7 days at 25°C, the water content of each crystal was measured.
The results are shown in Table 1.

**[Table 1]**

| Relative humidity (%RH) | Water content (%) | | | |
|---|---|---|---|---|
| | T-614α | T-614β | T-614γ | T-614hydrate |
| 22 | 0.30 | 0.54 | 0.28 | 4.09 |
| 51 | 1.29 | 0.25 | 0.33 | 4.15 |
| 75 | 2.88 | 0.21 | 0.52 | 4.08 |
| 93 | 3.62 | 0.18 | 0.65 | 4.03 |

Although the water content of T-614α increased with the rising of relative humidity, the water content of T-614β, T-614γ and T-614 hydrate, respectively was almost kept unchanged. T-614β, T-614γ and T-614 hydrate were stable under a high humidity condition.

### Examples

Condition of measurement for powder X-ray diffraction
Anti-cathode: Cu
Pipe voltage: 40kV
Pipe current: 25mA

### Example 1 Manufacturing of T-614β(1)

T-614α 1.00g was added to 40mL of 2-butanone, and the mixture was heated to dissolve. After cooling, precipitate crystal was filtered and air-dried to obtain 0.54g of T-614β.
IR (KBr) cm⁻¹: 3284, 1696
Water content: 0.07%

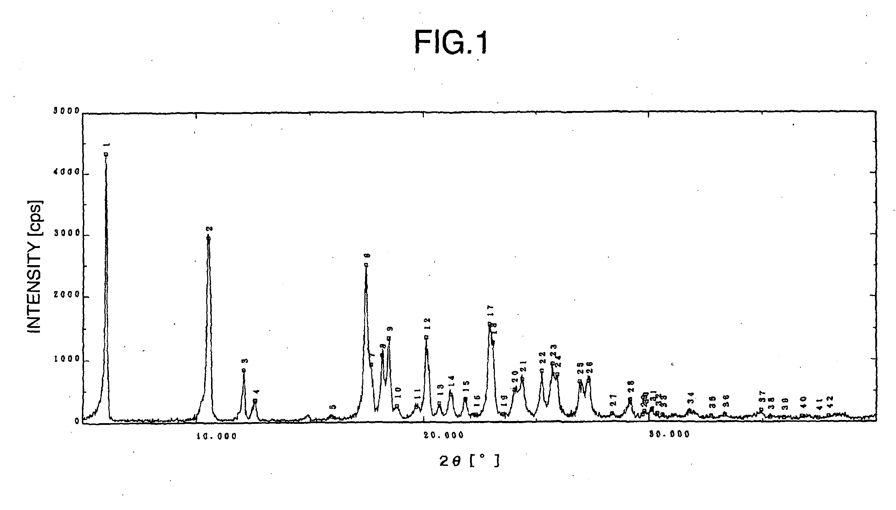

Powder X-ray diffraction pattern is shown in FIG. 1, the powder X-ray diffraction data are shown in Table 2.

**[Table 2]**

| 2θ(°) | d value (Å) | Relative intensity (%) |
|---|---|---|
| 6.02 | 14.67 | 100 |
| 10.52 | 8.40 | 68 |
| 17.42 | 5.09 | 58 |
| 18.44 | 4.81 | 31 |
| 20.10 | 4.41 | 31 |
| 22.94 | 3.87 | 36 |
| 23.08 | 3.85 | 30 |

### Example 2 Manufacturing of T-614β(2)

T-614α 1.00g was added to 500mL of toluene, and the mixture was heated to dissolve. After cooling, precipitate crystal was filtered and air-dried to obtain 0.91g of T-614β.
IR (KBr) cm⁻¹: 3284, 1696
Water content: 0.17%

Powder X-ray diffraction data are shown in Table 3. The powder X-ray diffraction pattern matched with that in Example 1.

**[Table 3]**

| 2θ(°) | d value (Å) | Relative intensity (%) |
|---|---|---|
| 5.96 | 14.82 | 45 |
| 10.50 | 8.42 | 100 |
| 17.40 | 5.09 | 65 |
| 18.36 | 4.83 | 53 |
| 20.06 | 4.42 | 29 |
| 22.92 | 3.88 | 43 |

### Example 3 Manufacturing of T-614β(3)

T-614α 0.50g was added to 10mL of toluene, and the mixture was stirred for 24 hours at 25°C. After filtration, the crystal was air-dried to obtain T-614β.
IR (KBr) cm⁻¹: 3284, 1696
Water content: 0.21%

Powder X-ray diffraction data are shown in Table 4. The powder X-ray diffraction pattern matched with Example 1.

**[Table 4]**

| 2θ(°) | d value (Å) | Relative intensity (%) |
|---|---|---|
| 6.00 | 14.72 | 43 |
| 10.56 | 8.37 | 100 |
| 17.42 | 5.09 | 58 |
| 18.38 | 4.82 | 51 |
| 20.16 | 4.40 | 32 |
| 23.06 | 3.85 | 47 |

### Example 4 Manufacturing of T-614β(4)

T-614γ 0.50g was added to 10mL of toluene, and the mixture was stirred for 1 hour at 25°C. After filtration of the mixture, the crystal was air-dried to obtain T-614β.
IR (KBr) cm⁻¹: 3284, 1696
Water content: 0.33%

Powder X-ray diffraction data are shown in Table 5. The powder X-ray diffraction pattern matched with Example 1.

**[Table 5]**

| 2θ(°) | d value (Å) | Relative intensity (%) |
|---|---|---|
| 6.02 | 14.67 | 40 |
| 10.56 | 8.37 | 100 |
| 17.42 | 5.09 | 65 |
| 18.42 | 4.81 | 49 |
| 20.16 | 4.40 | 30 |
| 23.00 | 3.86 | 45 |

### Example 5 Manufacturing of T-614β(5)

T-614α 0.3g was allowed to stand for 1 hour at 160°C. After cooling, T-614β was obtained.
IR (KBr) cm⁻¹: 3284, 1696
Water content: 0.19%

Powder X-ray diffraction data are shown in Table 6. The powder X-ray diffraction pattern matched with Example 1.

**[Table 6]**

| 2θ(°) | d value (Å) | Relative intensity (%) |
|---|---|---|
| 6.02 | 14.67 | 68 |
| 10.56 | 8.37 | 100 |
| 17.48 | 5.07 | 74 |
| 18.42 | 4.81 | 50 |
| 20.20 | 4.39 | 41 |
| 23.04 | 3.86 | 60 |

### Example 6 Manufacturing of T-614β(6)

T-614γ 0.3g was allowed to stand for 1 hour at 160°C. After cooling, T-614β was obtained.
IR (KBr) cm⁻¹: 3284, 1696
Water content: 0.23%

Powder X-ray diffraction data are shown in Table 7. The powder X-ray diffraction pattern matched with Example 1.

**[Table 7]**

| 2θ(°) | d value (Å) | Relative intensity (%) |
|---|---|---|
| 6.00 | 14.72 | 77 |
| 10.54 | 8.39 | 100 |
| 17.44 | 5.08 | 87 |
| 18.42 | 4.81 | 44 |
| 20.12 | 4.41 | 55 |
| 22.96 | 3.87 | 66 |

### Example 7 Manufacturing of T-614β(7)

T-614 hydrate 0.3g was allowed to stand for 1 hour at 160°C. After cooling, T-614β was obtained.
IR (KBr) cm⁻¹: 3284, 1695
Water content: 0.15%

Powder X-ray diffraction data are shown for Table 8. The powder X-ray diffraction pattern matched with Example 1.

**[Table 8]**

| 2θ(°) | d value (Å) | Relative intensity (%) |
|---|---|---|
| 5.98 | 14.77 | 48 |
| 10.52 | 8.40 | 100 |
| 17.36 | 5.10 | 65 |
| 18.32 | 4.84 | 51 |
| 20.10 | 4.41 | 34 |
| 23.04 | 3.86 | 50 |

### Example 8 Manufacturing of T-614γ

T-614 hydrate 15.0g was added to 600mL of acetonitrile, and the mixture was stirred for 24 hours at 25°C. After filtration of the mixture, the crystal was air-dried to obtain 13.6g of T-614γ.

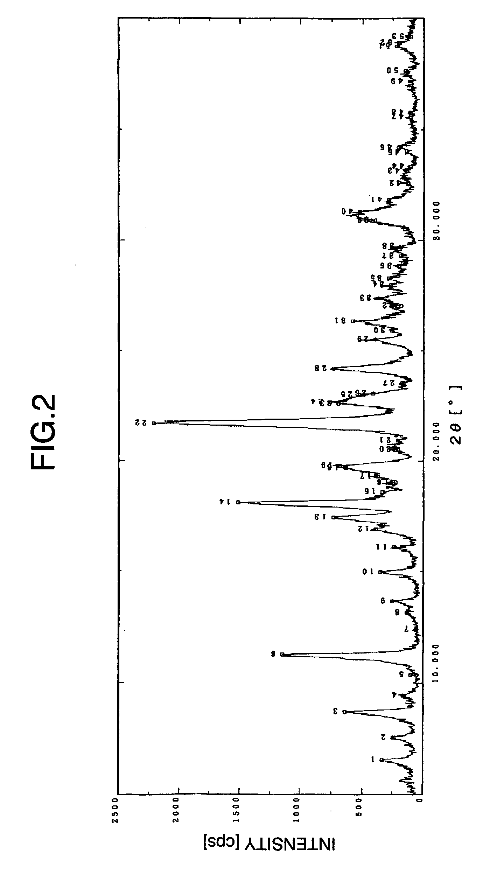

Powder X-ray diffraction pattern is shown in FIG. 2, powder X-ray diffraction data are shown in Table 9.
IR (KBr) cm⁻¹: 3279, 1683
Water content: 0.94%

**[Table 9]**

| 2θ(°) | d value (Å) | Relative intensity (%) |
|---|---|---|
| 11.28 | 7.84 | 52 |
| 17.44 | 5.08 | 33 |
| 18.12 | 4.89 | 69 |
| 19.78 | 4.48 | 31 |
| 21.70 | 4.09 | 100 |
| 22.68 | 3.92 | 35 |
| 24.18 | 3.68 | 33 |

### Example 9 Manufacturing of T-614 hydrate (1)

T-614α 15.0g was added to 450mL of water, and the mixture was stirred for 7 days at 25°C. After filtration of the mixture, the crystal was air-dried to obtain 15.1g of T-614 hydrate.
IR (KBr) cm⁻¹: 3614, 1681
Water content: 4.04%

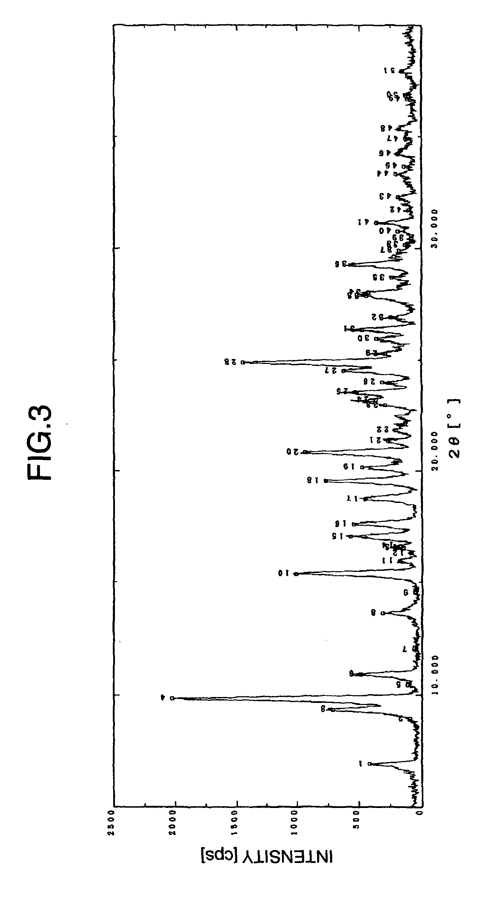

Powder X-ray diffraction pattern is shown in FIG. 3, powder X-ray diffraction data are shown in Table 10.

**[Table 10]**

| 2θ(°) | d value (Å) | Relative intensity (%) |
|---|---|---|
| 9.36 | 9.44 | 35 |
| 9.86 | 8.96 | 100 |
| 15.38 | 5.76 | 50 |
| 19.54 | 4.54 | 38 |
| 20.84 | 4.26 | 46 |
| 24.54 | 3.62 | 31 |
| 24.90 | 3.57 | 71 |

### Example 10 Manufacturing of T-614 hydrate (2)

T-614γ 0.50g was added to 10mL of water, and the mixture was stirred for 24 hours at 25°C. After filtration of the mixture, the crystal was air-dried to obtain T-614 hydrate.
IR (KBr) cm⁻¹: 3614, 1681
Water content: 4.49%

Powder X-ray diffraction data are shown in Table 11. The powder X-ray diffraction pattern matched with Example 9.

**[Table 11]**

| 2θ(°) | d value (Å) | Relative intensity (%) |
|---|---|---|
| 9.26 | 9.54 | 42 |
| 9.76 | 9.05 | 85 |
| 15.32 | 5.78 | 68 |
| 19.42 | 4.57 | 33 |
| 20.76 | 4.28 | 50 |
| 24.42 | 3.64 | 27 |
| 24.78 | 3.59 | 100 |

### Reference Example 1

T-614α was produced in the manner described as Example 5 in Japanese Patent Laid-Open No. 5-97840.

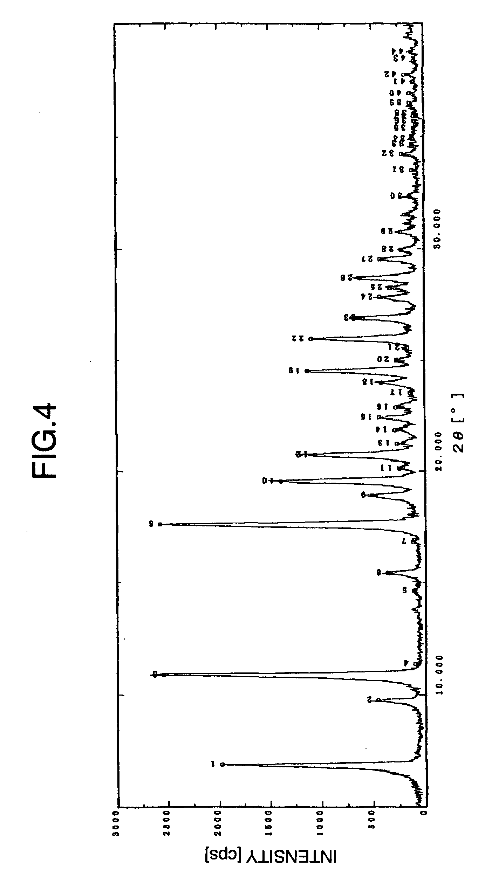

Powder X-ray diffraction pattern of T-614α is shown in FIG. 4, powder X-ray diffraction data are shown in Table 12.

**[Table 12]**

| 2θ(°) | d value (Å) | Relative intensity (%) |
|---|---|---|
| 6.90 | 12.80 | 77 |
| 10.90 | 8.11 | 99 |
| 17.60 | 5.04 | 100 |
| 19.56 | 4.53 | 54 |
| 20.74 | 4.28 | 41 |
| 24.52 | 3.63 | 44 |
| 25.98 | 3.43 | 42 |

### INDUSTRIAL APPLICABILITY

The crystal of T-614 having peaks at the positions of 6.00±0.05°, 10.54±0.05°, 17.42±0.08°, 18.39±0.08°, 20.13±0.10° and 23.01±0.10° on 2θ of diffraction angle in powder X-ray diffraction pattern, the crystal of T-614 having peaks at the positions of 11.28±0.05, 17.44±0.08°, 18.12±0.08°, 19.78±0.08°, 21.70±0.10°, 22.68±0.10° and 24.18±0.10° on 2θ of diffraction angle in powder X-ray diffraction pattern and the crystal of T-614 monohydrate having peaks at the positions of 9.31±0.05°, 9.81±0.05°, 15.35±0.08°, 19.48±0.08°, 20.80±0.10°, 24.48±0.10° and 24.84±0.10° on 2θ of diffraction angle in powder X-ray diffraction pattern are low hygroscopic, stable under a high humidity condition and are useful as drug substance of excellent anti-inflammatory agent.

## Claims

1. The crystal of N-[3-(formylamino)-4-oxo-6-phenoxy-4H-chromen-7-yl]methanesulfonamide having peaks at the positions of 6.00±0.05°, 10.54±0.05°, 17.42± 0.08°, 18.39±0.08°, 20.13±0.10° and 23.01±0.10° on 2θ of diffraction angle in powder X-ray diffraction pattern.

2. The crystal of N-[3-(formylamino)-4-oxo-6-phenoxy-4H-chromen-7-yl]methanesulfonamide having peaks at the positions of 11.28±0.05°, 17.44±0.08°, 18.12±0.08°, 19.78±0.08°, 21.70±0.10°, 22.68±0.10° and 24.18±0.10° on 2θ of diffraction angle in powder X-ray diffraction pattern.

3. The crystal of N-[3-(formylamino)-4-oxo-6-phenoxy-4H-chromen-7-yl]methanesulfonamide monohydrate having peaks at the positions of 9.31±0.05°, 9.81±0.05°, 15.35±0.08°, 19.48±0.08°, 20.80±0.10°, 24.48±0.10° and 24.84±0.10° on 2θ of diffraction angle in powder X-ray diffraction pattern.
